# EUROPEAN PATENT APPLICATION

(11) **EP 2 783 664 A1**
(43) Date of publication of application: **01.10.2014**
(21) Application number: 12852070.7
(22) Date of filing: 20.11.2012
(51) Int. Cl.: A61F 11/04, A61N 1/36

(54) **AUDITORY STIMULATION SYSTEM**

(30) Priority: 25.11.2011 ES 201131904
(71) Applicant: Slavutsky Joison, Victor Gustavoa, 08870 Sitges (Barcelona) (ES)
(72) Inventor: Slavutsky Joison, Victor Gustavoa, 08870 Sitges (Barcelona) (ES)
(74) Representative: Carpintero Lopez, Francisco
(86) International application number: PCT/ES2012/070807
(87) International publication number: WO 2013/076334

(57) **Abstract**

The invention relates to an auditory stimulation system with an external unit (1) receiving an acoustic signal and an internal unit (3) stimulating the auditory system of a user according to said acoustic signal, wherein the conversion means converting said acoustic signal into a stimulus signal are located in the external unit. The connection (2) between the internal unit and external unit is preferably made through the external auditory canal itself, the stimulus signal which is transmitted by said connection being an electrical signal, an airborne sound wave, a bone vibration, or a combination thereof.

## Description

### Field of the Invention

The present invention applies to the field of stimulation systems for the hearing impaired, particularly to auditory stimulation systems with elements that are surgically implanted in the user.

### Background of the Invention

Different types of auditory stimulation systems (or hearing aids) which help users with impaired auditory systems receive acoustic signals with higher quality by means of using electronic elements are known. Generally, all these systems comprise a microphone picking up an acoustic signal from the environment, electronic elements acting on said acoustic signal, and actuators transmitting the signal to the auditory system of the user. The various systems can be classified according to how the actuators stimulate the auditory system of the user.

Air conduction stimulation hearing aids were the first to be developed. The hearing aid receives sound through a microphone, converting the sound waves into electrical signals. An amplifier increases the volume of the electrical signal, said signal being subsequently converted back into sound waves by means of a loudspeaker located in the external auditory canal. The entire system is external, so it does not require surgical procedures for placement; in contrast, however, it does have serious limitations in terms of the intensity of the transmitted signal, therefore performing poorly in cases of severe and profound hearing loss. Additionally, sound pressure can cause the feeling of being stopped up, blocked and of hearing feedback, causing whistle-like auditory distortions.

Bone conduction stimulation systems by means of implant (BAHA, bone anchored hearing aid) are titanium devices placed by means of surgery in the skull bone, behind the pinna, as a result of complete titanium implant-bone integration at the molecular level. The hearing aid or sound processor receives the signal from the microphone and transmits the sound vibrations to the skull bone. The sound wave is then naturally propagated through the bone structure until the cochleae are stimulated. The external elements of the system are fixed to the user by means of the titanium screw itself which is surgically implanted in the bone. Nevertheless, BAHA systems still have limitations in terms of their performance, being indicated only for moderate and severe deafness.

A more advanced form of bone conduction stimulation are implants which act by vibrating the middle ear structures (VBS, Vibrant Sound Bridge). These implants look like the cochlear implants described below, but unlike them, the electrical signal generated by the microphone is transformed by a floating mass transducer (FMT) into vibration that is mechanically applied on the middle ear structures in a mechanical manner. The signal provided to the inner ear therefore has higher quality, further leaving the auditory canal open and the eardrum intact. However, despite this improvement, it still has limited performance, so it is not indicated for those with severe and profound deafness. It additionally requires completely preserving the middle ear structures. This last restriction was overcome by means of placing the FMT in the round window, although the limitation of the degree of deafness remains in this case.

Cochlear implants are transducers which transform acoustic signals into electrical signals stimulating the auditory nerve. To that end, they have the following parts:
- An external unit formed by a microphone receiving the acoustic signal, a processor operating on said signal, selecting the most useful sounds for speech compression and coding them again, and a transmitter sending the coded sounds to the internal unit
- The internal unit formed by a receiver-stimulator receiving the coded sounds from the transmitter and sending them in the form of electrical signals to electrodes that are introduced in the cochlea (inner ear) and stimulate functional nerve cells. These stimuli go through the auditory nerve to the brain which recognizes them as sounds producing the feeling of being able to hear. The receiver-stimulator is implanted in the mastoid bone, behind the pinna.
- A connection between the external unit and the internal unit. This connection is made through a winding, the internal unit having a magnet for receiving magnetic signal.

Cochlear implants allow treating cases of severe and profound hearing loss, but they have a series of drawbacks:
- The internal unit cannot be repaired in the event of system failures once implanted, so the receiver-stimulator must be surgically removed for repair should it fail.
- The system of transmitting the auditory signal by means of coils is unstable, causing transmitter sliding, insufficient magnet retention, etc.
- The magnet of the internal part prevents performing magnetic resonance imaging on users, surgical removal of the magnet being necessary in order to perform said imaging.
- High energy consumption due to the method of transmission between the external unit and the internal unit.
- Surgery for implantation thereof is complex.
- Although the auditory result is excellent, hearing is not natural.

Finally, to solve the issue of the naturalness of the sound that is heard, there are systems which combine electrical stimulation of the cochlear implant with acoustic stimulation (EAS, Electro Acoustic Stimulation). The hearing aid acoustically stimulates low frequencies, whereas the implant stimulates high frequencies, both being synchronized by one and the same processor distributing the auditory signal for the different frequencies. Sounds that are more natural can thus be heard, resulting in the ability to distinguish better in noisy environments and to hear music better. Nevertheless, the other drawbacks described for cochlear implants still exist.

There is therefore a need in the state of the art for a robust system which allows repair without surgery in the event of failure of auditory stimulation systems for severe and profound hearing loss.

### Description of the Invention

The present invention solves the problems described above by means of an auditory stimulation system in which the stimulator is located in the external part of the system to allow repair without surgical procedures. To allow placing the stimulator as such, the connection between the external unit and the internal unit is made by means of an electrical connection, furthermore eliminating the magnet and the winding, reducing system energy consumption and allowing magnetic resonance imaging to be performed on users.

The system of the present invention therefore comprises:
- An external unit placed outside the auditory system of the user and comprising a microphone receiving the acoustic signal and converting it into an electrical signal; and a stimulator converting the electrical signal of the microphone into a second electrical stimulus signal with the necessary characteristics for application in the auditory system of the user.
- An internal unit receiving the electrical stimulus signal and applying it in the auditory system of the user. It should be noted that conversion is therefore performed in the external unit, so the internal unit simply applies the electrical stimulus signal received, without performing any treatment thereon. To transmit the electrical stimulus signal between the external unit and the internal unit, the internal unit in turn comprises purely electrical connection means implanted in the external auditory canal. Said connection means are preferably formed by two cases:
   - An external case which is implanted behind the cartilaginous portion of the external auditory canal, leaving a slot (usually in an octahedral or cylindrical shape) as it has two opposing open faces.
   - A removable internal case which is fitted into the slot. More preferably, one of the ends of the internal case, particularly, the innermost end, is wedge-shaped.

Likewise, the internal case preferably comprises two compartments, in each of which there is introduced a plurality of electrical connections. In the lower compartment, said plurality of electrical connections corresponds to terminations of the stimulation means which can therefore be permanently implanted in the auditory system, whereas in the upper compartment there is introduced a prolongation of the external unit which can be connected and disconnected and removed without the need for surgical procedures.

The stimulus applied by the internal unit is preferably done through a plurality of electrodes acting directly on the cochlea of the user. Nevertheless, other options such as bone conduction stimulation, vibratory stimulation, acoustic stimulation or any combination thereof are contemplated.

In the event that several stimulation methods are combined, the system also comprises control means separating the frequencies to be stimulated by each method for the purpose of obtaining the most effective stimulation for each user. Again, given that the control means are located in the external unit, said control means can be accessed without surgery to perform frequency adjustment. The foregoing and other advantages of the invention will become apparent in view of the detailed description thereof.

### Brief Description of the Drawings

For the purpose of aiding to better understand the features of the invention according to a preferred practical embodiment thereof and to complement this description, the following illustrative and non-limiting drawings are attached as an integral part thereof:
Figure 1 shows a diagram of the auditory stimulation system according to a preferred embodiment of the invention and the placement thereof in the auditory system of a user.
Figure 2 shows a design of the connection means of the system according to a preferred embodiment thereof.
Figure 3 shows a diagram of the external unit of the system, as well as the elements forming it, according to a preferred embodiment thereof.
Figure 4 shows a diagram of the elements making up the system according to a preferred embodiment thereof in which electrical stimulation of the cochlea and bone conduction stimulation are combined.
Figure 5 shows a diagram of the elements making up the system according to a preferred embodiment thereof in which electrical-vibratory stimulation and electrical stimulation of the cochlea are combined.
Figure 6 shows a diagram of the elements making up the system according to a preferred embodiment thereof in which air conduction stimulation and electrical stimulation of the cochlea are combined.

### Detailed Description of the Invention

In this text, the term "comprises" and derivations thereof (such as "comprising", etc.) must not be understood in an exclusive manner, i.e., these terms must not be interpreted as excluding the possibility that everything described and defined may include more elements, steps, etc.

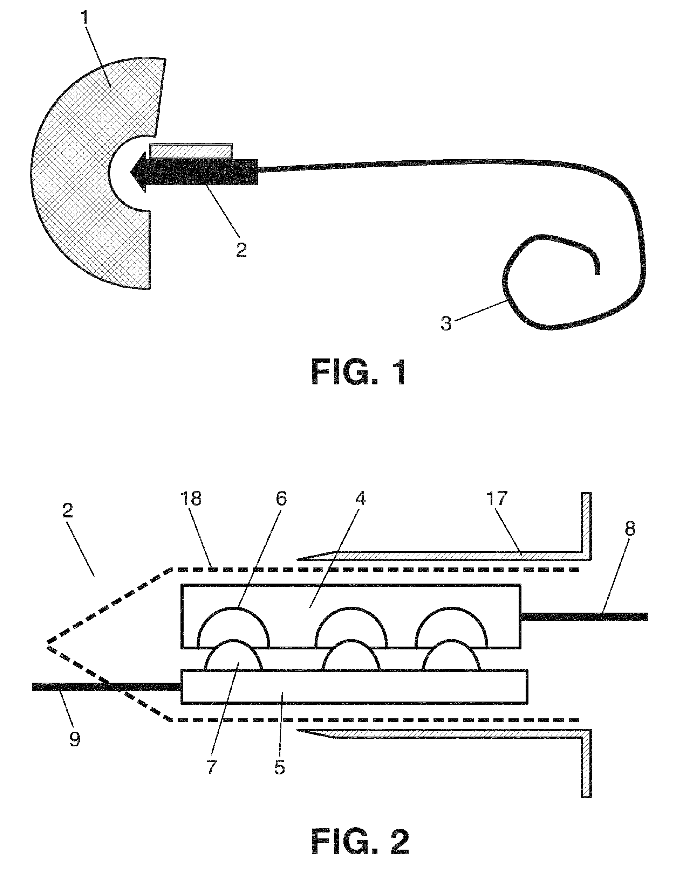

Figure 1 shows a preferred embodiment of the system of the invention already implanted in the auditory system of a user. The system comprises an external unit 1 installed behind the pinna which can be removed without surgery, and an internal unit 3 surgically implanted in the auditory system of the user and responsible for finally stimulating the cochlea by means of an electrode array. The internal unit 3 in turn comprises a connector 2 attaching the former to the external unit 1.

Figure 2 shows a more detailed diagram of the elements of said connector 2 formed by two cases:
- An external case 17 which is the part fixed to the bone at the mastoid area and has two free faces, thus demarcating a usually octahedral or cylindrical slot.
- An internal case 18 which is introduced in said slot, said internal case 18 being removable and containing interconnections between the external unit 1 and the internal unit 3.

A prolongation 8 in the form of a rigid shaft emerges from the external unit 1, at the end of which there are located a plurality of contacts 6 housed in an upper compartment 4 of the connector 2. Said plurality of contacts 6 is connected with a plurality of terminations 7 of the electrode guide 9. Said terminations 7 of the electrode guide 9 are introduced in a lower compartment 5 during surgical implantation of the device. This configuration of the connector allows mechanically removing the external unit 1 and its contact prolongation 8 without the need for surgery, removing it completely and allowing the adjustment or repair of all the elements of said external unit 1.

The leak-tight lower compartment 5 preferably has a wedge-shaped design at the innermost end, with a small hole to allow passage of the electrode guide 9. Titanium is the most appropriate material for manufacturing the connector given its proven osseointegration ability that allows, on one hand, fixing same in the bone, and on the other, passage of electric current once the prolongation of the external unit is in contact with the electrode guide of the internal unit. However, the use of other materials is obviously possible.

In one of the possible configurations of the connector 2, the two compartments are divided by a titanium sheet, such that the electrode guide 9 can be independently introduced and implanted while it allows removing and introducing the prolongation 8 attached to the processor 11 as many times as required. Said sheet comprises as many holes as terminations 7 in the electrode guide 9. Therefore, after introducing the prolongation 8, the crescent-shaped contacts 6 would be introduced in the holes and the electrical connection would be established. Nevertheless, it should be noted that this is only one of the possible ways to establish said electrical connection within the connector and that other configurations can be used within the object of the present invention as claimed.

In terms of the implantation of the system, surgery starts with the placement of the connector 2 immediately behind the pinna. The electrode guide 9 is introduced through the lower compartment 5, ending in a wedge shape, said electrode guide 9 exiting through the hole at the inner end thereof, for finally introducing the electrode array in the cochlea. The outer end of the electrode guide 9 ending in an oar blade shape completely closes the lower compartment 5 in the innermost portion thereof, leaving the crescent-shaped terminations 7 facing the upper compartment 4. The prolongation 8 of the external unit 1 in the form of a shaft, comprising contacts 6 at its end, slides through the upper compartment 4 until reaching the terminations 7 of the electrode guide 9. The contacts 6 have an inverse shape of the crescent shape of the terminations 7 of the electrode guide 9, contact and signal transmission between the external unit 1 and the internal unit 3 thus occurring.

The connector 2 allows being used for both the left ear and the right ear. To that end, it is sufficient to rotate said connector 2 180° such that the upper compartment and the lower compartment switch positions.

The length of the connector 2 is adjusted by means of the relative sliding of the external case 17 and the internal case 18. Likewise, the prolongation 8 of the external unit 1 is also adjustable in length. Said adjustment is achieved by introducing the prolongation 8 in or removing it from the capsule of the external unit 1, and if necessary, orienting the contacts by means of rotating same. System flexibility and length adjustment allow correct electrical signal transmission.

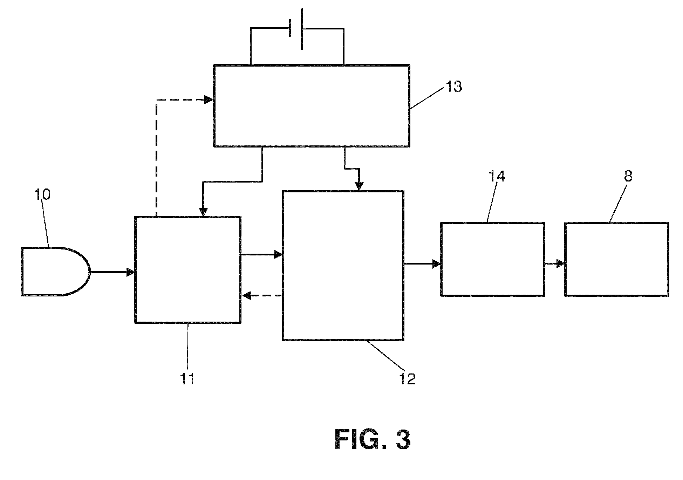

Figure 3 shows the elements which are part of the external unit:
- Microphone 10 capturing acoustic signals and converting them into electrical signals to be used by the rest of the system.
- Processor 11 responsible for polarizing the microphone 10 for operation, amplifying and processing the electrical signal received from said microphone 10 and determining the most ideal parameters to perform stimulation. In the case of combining several stimulation methods as detailed below, the processor determines which frequencies are stimulated by each stimulation method.
- Stimulator control 12. The purpose of this element is to generate electrical stimulus signals, i.e., impulses applied to the auditory nerve. The stimuli can be a current stimulus or a voltage stimulus having variable amplitude and duration. The stimulation parameters are determined by the processor 11 depending on the received signal and on the processing thereof.
- Energy regulator 13. Different operating voltages from a single power supply source, usually a battery, are required for processing, control and stimulation. Generating said voltages requires the energy regulator 13 formed by switching regulators optimizing efficiency and allowing different voltage levels, as required, separately optimizing all the elements of the system. The regulator 13 is controlled by the processor 11 which has a control to determine the best operating conditions depending on the moment and/or on the signal captured by the microphone 10.
- Protective capacitors 14. They are DC decoupling capacitors that prevent stimulator malfunction from being able to damage the nerve and/or electrodes. This situation may arise if an element of the stimulator is damaged and the ground power supply through the electrodes short circuit. The capacitor of the protective capacitor 14 limits the load transmitted to the internal unit 3, which prevents damaging said internal unit 3 and harming the user in the event of failure.

In the case of combining several stimulation methods, the processor 11 codes the electrical signal required for high frequencies and the acoustic signal required for low frequencies. This is achieved with a previous audiogram indicating the frequency up to which the patient has residual hearing, allowing acoustic stimulation; the processor is programmed with this data to stimulate up to a specific frequency acoustically, for example, and the rest of the frequencies electrically.

Performing an audiometry provides information about the affected frequencies and the degree of hearing loss and this allows programming the hearing aid by air conduction so that the affected frequencies can be stimulated with the necessary intensity and does not result in an acoustic stimulation that is uncomfortable for the patient; however, programming the processor for electrical stimulation does not exactly correspond with the audiogram, rather it corresponds approximately with the position occupied by the electrodes in the cochlea. Those electrodes which are located at the start of the cochlea stimulate the highest frequencies, and the electrodes that are progressively located closer to the tip or apex stimulate the lowest frequencies passing through the entire medium frequency range. This is called tonotopic stimulation, and the different frequencies are stimulated depending on the position, number and insertion depth of the electrodes.

The internal unit 3 stimulates the cochlea by applying the electrical stimulus signal received through the connector 2. To that end, the internal unit 3 has a guide with a plurality of electrodes implanted in the cochlea, each of them connected at the opposite end to a termination 7 which is housed in the lower compartment 5 of the connector 2. There is also an additional reference electrode termination 7.

The reference electrode is an electrode located outside the cochlea (extracochlear) allowing a specific type of electrical stimulation called monopolar stimulation. Electrical stimulation requires an active electrode and another inactive or reference electrode, such that an electric field is created between the two electrodes to stimulate a specific area of the cochlea. The farther away the active electrode is located, the more stimulation field is generated. If a specific area of the cochlea is to be stimulated, bipolar stimulation between two electrodes located close to one another in the cochlea is used, in which one of the electrodes is inactivated to act as a reference electrode and the other electrode is the active electrode. Bipolar stimulation does not require the electrodes to be contiguous, being able to leave out one or several electrodes between the two electrodes responsible for stimulation. A specific point can thus be stimulated, improved electrical response being obtained in that area, but with higher energy consumption. Bipolar stimulation does not take the reference electrode into account.

It should be noted that it is possible to use configurations with several reference electrodes, for example, one electrode that is part of the stimulator and another electrode located remotely on the outside, by means of a cable, combining the electrical stimulation using one electrode or the other. Likewise, the specific position of the reference electrode or electrodes and of the remaining electrodes depends on the stimulation strategy, and there can therefore be different electrode configurations within the object of the present invention.

The stimulator 12 is part of the external unit 1 allowing easy access for control or repair, eliminating the need for magnets and coils and reducing total device consumption. The system is implanted by means of EMA (endomeatal approach) surgery through the natural orifice of the external auditory canal corresponding to the anatomical area of the mastoid process, where the connector 2 is positioned and the external unit 1 is also fastened. This differs greatly from the implants of the state of the art implanted by means of surgery through the mastoid process and with the stimulator 12 located in the inner unit 3, because in that case said bone is removed and the large space that it requires makes it necessary to transmit the auditory signal electromagnetically. In addition to all the described advantages, given that the design of the system of the invention allows implantation by means of EMA surgery, surgical risks, infections, and other complications are reduced.

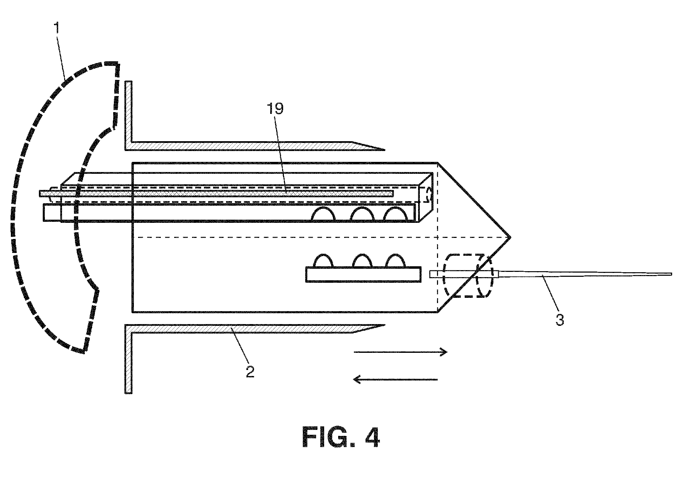

Figure 4 shows a system in which the electrical stimulation described in detail up until now is combined with bone conduction stimulation, in which the connector 2 acts as a bone stimulator, replacing the conventional BAHA screw. To that end, it has a vibrating wafer 19 which is introduced in the upper compartment 4 of the connector 2. The processor 11 determines the frequencies that must be stimulated by each system, bone conduction stimulation normally being used for low frequencies and electrical stimulation for high frequencies.

Even if bone conduction stimulation alone is used in the present system, the position of the connector 2 closer to the cochlea allows better stimulation than conventional BAHA systems. Nevertheless, it should be noted that bone conduction stimulation alone would only be valid for moderate to severe hearing loss. With respect to other bone conduction stimulations, in the case of bone conduction stimulation alone according to the invention a lower risk of infection is further achieved due to the absence of hair follicles. Stimulation is percutaneous and not transcutaneous as in conventional bone conduction hearing aids. Another advantage with respect to BAHA is that osseointegration of the connector 2 is not strictly necessary since vibration is transmitted by contiguity as a result of said connector being in direct contact with the bone surface of the mastoid process, milling not being necessary, unlike in BAHA which requires placing a screw in the skull for fixing and fastening. The connector 2 does not need to be fastened because it is located between the bone surface of the mastoid process and the cartilaginous structure of the pinna positioning it. However, a bone bed for the connector 2 can be milled with a supplement if a more intense signal needs to be transmitted, depending on the state of bone conduction of the patient.

Other arrangements of the vibrating wafer and other ways of supplementing the internal case are also contemplated to achieve a more direct bone vibration transmission, both the internal case and the vibrating wafer being able to be in direct contact with the bone.

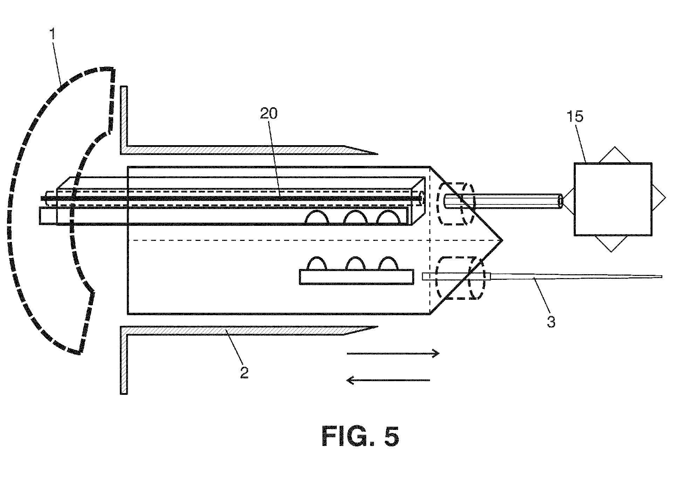

Figure 5 shows a system in which electrical stimulation is combined with electrical-vibratory stimulation. In this case, the processor 11 determines which frequencies are electrically stimulated and which frequencies are stimulated by a VBS 15. In a preferred option, frequencies with moderate-severe hearing loss are stimulated in an electrical-vibratory manner and frequencies with profound hearing loss are stimulated electrically. Despite also being bone conduction stimulation, vibration of the VBS 15 is directly transmitted to one of the ossicles (the incus, where it is fixed). Since it is a direct stimulation of a middle ear component, perception is more natural than with BAHA stimulation.

There are two preferred options for connection between the processor 11 and the VBS 15:
- By means of coils. To that end the stimulator 12 comprises a cable connecting with the FMT. There are two guides, one for electrical stimulation and the other for stimulation through vibration. Signal transmission by means of coils makes cranial positioning necessary and requires more extensive surgery, but insertion of the electrode in the cochlea and fixing of the FMT in the tympanic cavity are carried out with endomeatal (EMA) surgery.
- Direct vibratory signal transmission. Connection of the FMT with the processor 11 located in the external unit 1 is established through the connector 2. Contact with the FMT is usually established by means of a tube 20 in the upper compartment 4.

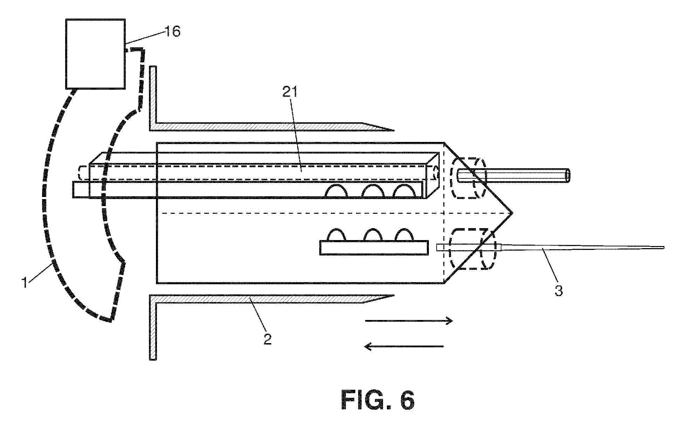

Finally, Figure 6 shows a system in which electrical stimulation is combined with air conduction stimulation by means of a hearing aid 16. Said hearing aid 16 can be controlled independently or through the processor 11 of the external unit 1. The connector 2 comprises a tube 21 to provide an exit for sound pressure and for directing same to the external auditory canal. For the preferred case in which the hearing aid 16 is controlled by the processor 11, therefore allowing greater control over the operating conditions of said hearing aid, there are two preferred options for communication between the processor 11 and the hearing aid 16:
- A polyethylene tube coming out of the processor 11, carrying an acoustic signal to a mold housed in the external auditory canal.
- A titanium tube integrated in the connector 2 itself with an outlet to the auditory canal at its inner end and a connection to the processor 11 at its outer end.

In another preferred embodiment, the system comprises a tube, preferably a titanium tube, independent of the connector. The tube is housed in a groove made in the rear wall of the bone portion of the auditory canal and comprises two spaces or compartments:
- A first space for the passage of the electrode guide.
- A second space for the passage of sound pressure either directly or through a polyethylene tube or a tube made of another equivalent material.

Likewise, any other combination of the described forms of stimulus, including bone conduction stimulation and air conduction stimulation; bone conduction, air conduction and electrical stimulation, etc., can be made.

In view of this description and drawings, the person skilled in the art will understand that the invention has been described according to several preferred embodiments thereof but that multiple variations can be introduced in said preferred embodiments without departing from the scope of the invention as claimed.

## Claims

1. Auditory stimulation system comprising:
- an external unit (1) suitable for being placed outside an auditory system of a user, said external unit comprising a microphone (10) suitable for receiving an acoustic signal and generating an electrical signal;
- an internal unit (3) suitable for being placed in the auditory system, said internal unit (3) comprising stimulation means suitable for applying a stimulus emulating the acoustic signal to said auditory system, and connection means (2) for connection with the external unit (1);
**characterized in that** the external unit (1) further comprises conversion means (12) suitable for converting the electrical signal of the microphone (10) into an electrical stimulus signal, **in that** the connection means (2) are electrical connection means suitable for transmitting said electrical stimulus signal, and **in that** the stimulation means apply said electrical stimulus signal in the auditory system of the user.

2. Auditory stimulation system according to claim 1, **characterized in that** the internal unit (3) is suitable for being implanted in an external auditory canal and on an outer surface of the mastoid process.

3. Auditory stimulation system according to claim 2, **characterized in that** the connection means (2) comprise:
- an external case (17) with two opposing open faces which is implanted behind the external auditory canal at the retroauricular sulcus level and demarcates a slot;
- a removable internal case (18) which is fitted into said slot.

4. Auditory stimulation system according to claim 3, **characterized in that** the internal case (18) comprises a wedge-shaped end, said end being located in the innermost portion (3).

5. Auditory stimulation system according to any of claims 2 to 4, **characterized in that** the connection means (2) comprise:
- a first compartment (5) comprising a first plurality of electrical connections (7) connected to terminations of the stimulation means of the internal unit (3);
- a second compartment (4) in which there is introduced a second plurality of electrical connections (6) located in a prolongation of the external unit (1); said second plurality of electrical connections (6) being suitable for being connected to the first plurality of electrical connections (7), and said prolongation being able to be removed from the auditory system of the user.

6. Auditory stimulation system according to any of claims 3 to 5, **characterized in that** the connection means (2) of the internal unit (3) are fixed to an external bone surface of the mastoid process and to a bone portion of the external auditory canal.

7. Auditory stimulation system according to any of the preceding claims, **characterized in that** the stimulation means of the internal unit (3) comprises an electrode array suitable for electrically stimulating the cochlea.

8. Auditory stimulation system according to any of the preceding claims, **characterized in that** the stimulation means of the internal unit (3) comprise bone conduction stimulation means.

9. Auditory stimulation system according to any of the preceding claims, **characterized in that** the stimulation means of the internal unit comprise electrical-vibratory stimulation means.

10. Auditory stimulation system according to any of the preceding claims, **characterized in that** the stimulation means of the internal unit (3) comprise air conduction acoustic stimulation means.

11. Auditory stimulation system according to any of the preceding claims, **characterized in that** the stimulation means of the internal unit (3) comprise one or several means including: electrodes suitable for electrically stimulating the cochlea, bone conduction stimulation means, electrical-vibratory stimulation means, and air conduction acoustic stimulation means; and **in that** the external unit comprises control means (11) determining frequencies to be stimulated by each stimulation means.

12. Auditory stimulation system according to any of the preceding claims, **characterized in that** the external unit (1) further comprises protective decoupling capacitors (14) located between the conversion means (12) and the connection means (2).

13. Auditory stimulation system according to any of the preceding claims, **characterized in that** it further comprises a tube independent of the connector (2), said tube being housed in a groove made in the rear wall of the bone portion of the auditory canal, and **in that** said tube comprises:
- a first space facilitating the passage of the electrode guide into the cochlea;
- a second space guiding sound pressure of an acoustic stimulation.

14. Auditory stimulation system according to claim 13, **characterized in that** the second space comprises a polyethylene tube for guiding said sound pressure.
